# EUROPEAN PATENT APPLICATION

(11) **EP 4 481 747 A1**
(43) Date of publication of application: **25.12.2024**
(21) Application number: 23781327.4
(22) Date of filing: 29.03.2023
(51) Int. Cl.: G16B 40/20, G06T 7/00, G06N 20/00, G06N 3/08, G16H 50/20

(54) **METHOD FOR TRAINING MACHINE LEARNING MODEL TO ANALYZE IMMUNOHISTOCHEMICALLY STAINED IMAGES AND COMPUTING SYSTEM PERFORMING SAME**

(30) Priority: 31.03.2022 KR 20220040558
(71) Applicant: Deep Bio Inc., Seoul 08380 (KR)
(72) Inventor: KWAK, Tae Yeong, Seoul 05119 (KR); YANG, Hyeon Seok, Seoul 08760 (KR); KIM, Sun Woo, Seongnam-si, Gyeonggi-do 13599 (KR)
(74) Representative: Flügel Preissner Schober Seidel
(86) International application number: PCT/KR2023/004145
(87) International publication number: WO 2023/191472

(57) **Abstract**

Disclosed herein are a method for training a machine learning model and a computing system performing same, wherein the machine learning model is trained to analyze biological tissue slide images stained by a immunohistochemical staining method for staining tissues expressing specific biomarkers and thus can be used to further elaborately analyze expression levels of biomarkers, etc., whereby a determination can be made on pathological specimen images by analyzing expression levels of biomarkers, etc. Provided according to one aspect of the invention is a method and system for training a machine learning model using training data, corresponding to immunohistochemically stained images, generated from multiple feature vectors calculated based on various staining intensity criteria.

## Description

### Technical Field

The present disclosure relates to a method for training a machine learning model for analyzing an immunohistochemically stained image and a computing system for performing the same. More specifically, the present disclosure relates to a method for training a machine learning model which is used to precisely analyze the expression level of a biomarker by analyzing a biological tissue slide image stained by an immunohistochemical staining method which stains tissues in which a specific biomarker is expressed, and for performing a determination on a pathological specimen image, such as analyzing the expression level of the biomarker, using the trained machine learning model, and a computing system for performing the same.

### Background Art

As the average life expectancy of modern people increases, the incidence of serious diseases such as cancer is also increasing, and measuring the degree to which specific biomarkers are expressed in biological tissues is a very important factor in predicting the prognosis or determining treatment methods for patients with serious diseases such as cancer. Although next generation sequencing (NGS) and other methods have recently been in the spotlight as a method for measuring the expression level of biomarker in biological tissues, the method still mainly used in clinical settings at medical institutions is to visually observe tissue slides stained with an immunohistochemistry (IHC) staining method.

The IHC staining method stains tissues using a dye in a form which combines antibodies targeting a specific biomarker with a chromogen such as diaminobenzidine (DAB), allowing the expression level of the biomarker to be visually recognized. At this time, hematoxylin is used as a control stain to indicate cells which do not express the biomarker.

The reading of immunohistochemically stained tissues is generally done by considering the intensity of the staining and the proportion of tissue stained. At this time, the staining intensity is usually divided into four levels of 'almost none', 'weak', 'moderate', and 'strong'. Depending on the biomarker, staining of the tissue may be recognized as occurring when only a portion of the cell nucleus is stained, or when the entire cell membrane is stained. In general, it is common to determine the target area (e.g., cancer lesion) for measuring the expression level of the biomarker, and then select and read the target for confirming the actual expression of the biomarker, such as cancer cells or immune cells.

Meanwhile, the reading rules for immunohistochemically stained tissues vary depending on the target biomarker to be measured and the applicable organ/disease, and this is because the expression pattern of each biomarker is different, and the expression level for the same biomarker may vary depending on the applicable organ/disease. In the case of specific IHC staining methods that measure the expression levels of estrogen receptor (ER) and progesterone receptor (PR), the total score obtained by calculating the proportion of stained tissue (proportion score) from 0 to 5 points and the staining intensity (intensity score) from 0 to 3 points, respectively, and adding them together is used, and in the case of specific IHC staining methods that measure the expression level of human epidermal growth factor receptor 2 (HER2), the score is determined as 0, 1+, 2+, or 3+ based on the proportion of cancer cells determined to be stained, taking into account the staining intensity and staining degree of the cell membrane. In some cases, when the expression rate of PD-L1 of tumor-infiltrating immune cells is 1% or higher, it is determined to be expressed, and even in this case, the cell membrane of the immune cells must be stained with an intensity higher than an appropriate level.

As such, reading immunohistochemically stained tissues by considering the staining intensity and the proportion of stained tissue is very difficult for the following reasons, the inter-reader agreement is poor, and the reproducibility between individual readers is also poor. First, the criteria for distinguishing staining intensity are unclear, and in particular, the boundary between weak and moderate is often unclear. Therefore, the reading results may vary from day to day depending on the condition of the reader. Second, there are many cases where it is difficult to determine whether the tissue is stained. In particular, when staining is determined by considering the staining intensity, it is not easy to determine whether cells with cell membranes that are some strongly and some moderately or weakly stained are stained.

Meanwhile, as image analysis technology advances, attempts are being made to apply and commercialize machine learning technology in various tasks in the medical field. In the field of pathology diagnosis, instead of the existing method in which a pathologist diagnoses by examining and reading stained tissue slides at high magnification through an optical microscope, a method in which tissue slides are converted into high-resolution digital images using a digital slide scanner and then the pathologist reads the images on a computer monitor is gradually becoming practical, and furthermore, products are emerging that allow pathologists to make faster and more accurate diagnoses by referring to the results of image analysis using machine learning technology. The method of diagnosing cancer by applying image analysis deep learning technology to whole-slide images (WSI), which are images created by scanning the entire biological tissue slide, has already reached the commercial level, and there are products that have been approved as medical devices domestically and internationally for some types of cancer, such as prostate cancer. These products perform functions such as determining the presence or absence of cancer lesions or visualizing the location of detected lesions by analyzing WSIs created from stained slides.

In addition, with the recent development of digital pathology and the significant advancement of computer-based image analysis technologies such as image processing, machine learning, and deep learning, software that automatically measures the expression level of biomarkers by analyzing digital images scanned from immunohistochemically stained slides has emerged and is continuously being advanced, and if the expression level of biomarkers is automatically measured by software, the problems of inter-reader agreement and individual reader reproducibility may be solved.

However, since the software produces results based on reading rules developed for an environment in which people measure and read using an optical microscope, detailed analysis of the expression level of biomarker is impossible. For example, when using a software, staining intensity could be distinguished in much more detail than just "almost none", "weak", "moderate", and "strong", and the staining level in each cell could even be quantitatively determined, but such analysis is currently impossible with the system. Therefore, in order to make more precise decisions about patient treatment based on the expression level of biomarker, a new interpretation method that may perform detailed analysis of the expression level of biomarker is needed. In addition, a technical idea is required that may perform determination on pathological specimen images, such as analyzing the expression level of biomarker by training a machine learning model that is used to precisely analyze the expression level of biomarker applying this to training a machine learning model.

### Disclosure of the Invention

### Technical Goals

A technical object to be achieved by the present disclosure is to provide a method and system capable of precisely analyzing the expression level of a biomarker by analyzing an immunohistochemically stained tissue slide using machine learning. In addition, an object of the present disclosure is to provide a method and system capable of performing determination on various pathological specimen images, such as training a machine learning model used to precisely analyze the expression level of biomarkers and analyzing the expression level of biomarkers through the machine learning model.

### Technical Solutions

According to an aspect of the present disclosure, there is provided a method for training a machine learning model, the method including: generating, by a machine learning model training system, a training data set including M pieces of individual training data (where M is a natural number of 2 or more); and training, by the machine learning model training system, the machine learning model based on the training data set, wherein the generating of the training data set including the M pieces of individual training data includes, for all integers m where 1<=m<=M, generating m^{th} training data to be included in the training data set, wherein the generating of the m^{th} training data includes: acquiring an m^{th} immunohistochemically stained image, wherein the m^{th} immunohistochemically stained image includes an area corresponding to an immunohistochemically stained tissue stained by an immunohistochemistry (IHC) staining method for staining a predetermined target biomarker; calculating a staining intensity by immunohistochemical staining for each pixel of the m^{th} immunohistochemically stained image; for all integers n where 1<=n<=N (where N is an integer of 2 or more), generating an n^{th} feature vector of the m^{th} immunohistochemically stained image based on the staining intensity for each pixel of the m^{th} immunohistochemically stained image and a predetermined n^{th} staining intensity reference value; and generating the m^{th} training data based on the first staining intensity reference value to the N^{th} staining intensity reference value and the first feature vector to the N^{th} feature vector of the m^{th} immunohistochemically stained image, and wherein the generating of the n^{th} feature vector of the m^{th} immunohistochemically stained image based on the staining intensity for each pixel of the m^{th} immunohistochemically stained image and the predetermined n^{th} staining intensity reference value includes generating an n^{th} binarized image corresponding to the m^{th} immunohistochemically stained image by comparing the staining intensity for each pixel of the m^{th} immunohistochemically stained image with the n^{th} staining intensity reference value, wherein the n^{th} binarized image is an image divided into an area having a staining intensity greater than the n^{th} staining intensity reference value and an area not having the same, and generating the n^{th} feature vector of the m^{th} immunohistochemically stained image based on the n^{th} binarized image corresponding to the m^{th} immunohistochemically stained image.

In an embodiment, for all integers i where 1<=i<=(N-1), the i^{th} staining intensity reference value may be smaller than the (i+1)^{th} staining intensity reference value.

In an embodiment, the acquiring of the m^{th} immunohistochemically stained image may include acquiring an m^{th} original pathological image generated by scanning a pathological slide stained with a dye in which an immunohistochemical staining reagent and a counterstaining reagent are mixed; and separating an immunohistochemically stained part stained with the immunohistochemical staining reagent and a counterstained part stained with the counterstaining reagent from the m^{th} original pathological image, thereby generating the m^{th} immunohistochemically stained image corresponding to the m^{th} original pathological image and an m^{th} counterstained image corresponding to the m^{th} original pathological image.

In an embodiment, the generating of the n^{th} feature vector of the m^{th} immunohistochemically stained image based on the n^{th} binarized image corresponding to the m^{th} immunohistochemically stained image may include generating the n^{th} feature vector of the m^{th} immunohistochemically stained image, in which each of at least one calculated value calculated based on the n^{th} binarized image corresponding to the m^{th} immunohistochemically stained image and the m^{th} counterstained image is a component of the vector, wherein the at least one calculated value includes at least one of a proportion of stained cell tissue, a proportion of cells with stained cell membranes, and a proportion of cells with stained cell nuclei.

In an embodiment, the generating of the m^{th} training data based on the first staining intensity reference value to N^{th} staining intensity reference value and the first feature vector to N^{th} feature vector of the m^{th} immunohistochemically stained image may include generating the m^{th} training data, which includes a pair of the first staining intensity reference value and the first feature vector to a pair of the N^{th} staining intensity reference value and the N^{th} feature vector, and is labeled with a human epidermal growth factor receptor 2 (HER2) expression score.

In an embodiment, the generating of the m^{th} training data based on the first staining intensity reference value to the N^{th} staining intensity reference value and the first feature vector to the N^{th} feature vector of the m^{th} immunohistochemically stained image may include generating the m^{th} training data, which includes a pair of the first staining intensity reference value and a first feature vector difference value to a pair of the (N-1)^{th} staining intensity reference value and an (N-1)^{th} feature vector difference value, and is labeled with an estrogen receptor (ER) or progesterone receptor (PR) expression score, wherein, for all integers i where 1<=i<=(N-1), the i^{th} feature vector difference value is a difference value between the (i+1)^{th} feature vector and the i^{th} feature vector.

According to another aspect of the present disclosure, there is provided a method for providing a determination result for a predetermined determination target pathological specimen through a machine learning model trained by the above-described method, the method including, acquiring, by a computing system, a determination target immunohistochemically stained image, wherein the determination target immunohistochemically stained image includes an area corresponding to an immunohistochemically stained tissue of the determination target pathological specimen stained by the IHC staining method; calculating, by the computing system, a staining intensity by immunohistochemical staining for each pixel of the determination target immunohistochemically stained image; for all integers n where 1<=n<=N, generating, by the computing system, an n^{th} feature vector of the determination target immunohistochemically stained image based on the staining intensity for each pixel of the determination target immunohistochemically stained image and the n^{th} staining intensity reference value; and generating, by the computing system, input data based on the first staining intensity reference value to the N^{th} staining intensity reference value and the first feature vector to the N^{th} feature vector of the determination target immunohistochemically stained image; and outputting, by the computing system, a determination result for the determination target pathological specimen determined by the machine learning model based on the input data, wherein the generating of the n^{th} feature vector of the determination target immunohistochemically stained image based on the staining intensity for each pixel of the determination target immunohistochemically stained image and the n^{th} staining intensity reference value includes generating an n^{th} binarized image corresponding to the determination target immunohistochemically stained image by comparing the staining intensity for each pixel of the determination target immunohistochemically stained image with the n^{th} staining intensity reference value, wherein the n^{th} binarized image is an image divided into an area having a staining intensity greater than the n^{th} staining intensity reference value and an area not having the same, and generating the n^{th} feature vector of the determination target immunohistochemically stained image based on the n^{th} binarized image corresponding to the determination target immunohistochemically stained image.

According to another aspect of the present disclosure, there is provided a computer program recorded on a non-transitory medium for performing the above-described method which is installed in a data processing device.

According to another aspect of the present disclosure, there is provided a non-transitory computer-readable recording medium in which a computer program for performing the above-described method.

According to another aspect of the present disclosure, there is provided a machine learning model training system including a processor; and a memory storing a computer program, wherein the computer program, when executed by the processor, causes the machine learning model training system to perform a machine learning model training method, wherein the machine learning model training method includes, generating, by the machine learning model training system, a training data set including M pieces of individual training data (where M is a natural number of 2 or more); and training, by the machine learning model training system, the machine learning model based on the training data set, wherein the generating of the training data set including M pieces of individual training data includes, for all integers m where 1<=m<=M, generating m^{th} training data to be included in the training data set, wherein the generating of the m^{th} training data includes acquiring an m^{th} immunohistochemically stained image, wherein the m^{th} immunohistochemically stained image includes an area corresponding to an immunohistochemically stained tissue stained by an IHC staining method for staining a predetermined target biomarker; calculating a staining intensity by immunohistochemical staining for each pixel of the m^{th} immunohistochemically stained image; for all integers n where 1<=n<=N (where N is an integer of 2 or more), generating an n^{th} feature vector of the m^{th} immunohistochemically stained image based on the staining intensity for each pixel of the m^{th} immunohistochemically stained image and a predetermined n^{th} staining intensity reference value; and generating the m^{th} training data based on the first staining intensity reference value to the N^{th} staining intensity reference value and the first feature vector to the N^{th} feature vector of the m^{th} immunohistochemically stained image, and wherein the generating of the n^{th} feature vector of the m^{th} immunohistochemically stained image based on the staining intensity for each pixel of the m^{th} immunohistochemically stained image and the predetermined n^{th} staining intensity reference value includes generating an n^{th} binarized image corresponding to the m^{th} immunohistochemically stained image by comparing the staining intensity for each pixel of the m^{th} immunohistochemically stained image with the n^{th} staining intensity reference value, wherein the n^{th} binarized image is an image divided into an area having a staining intensity greater than the n^{th} staining intensity reference value and an area not having the same, and generating the n^{th} feature vector of the m^{th} immunohistochemically stained image based on the n^{th} binarized image corresponding to the m^{th} immunohistochemically stained image.

According to another aspect of the present disclosure, there is provided a determination result providing system for a pathological specimen, the system including a processor; and a memory storing a computer program, wherein the computer program, when executed by the processor, causes the determination result providing system to perform a method for providing a determination result for a predetermined determination target pathological specimen through a machine learning model trained by the above-described method, wherein the method for providing a determination result includes, acquiring, by the determination result providing system, a determination target immunohistochemically stained image, wherein the determination target immunohistochemically stained image includes an area corresponding to an immunohistochemically stained tissue of the determination target pathological specimen stained by the IHC staining method; calculating, by the determination result providing system, a staining intensity by immunohistochemical staining for each pixel of the determination target immunohistochemically stained image; for all integers n where 1<=n<=N, generating, by the determination result providing system, an n^{th} feature vector of the determination target immunohistochemically stained image based on the staining intensity for each pixel of the determination target immunohistochemically stained image and the n^{th} staining intensity reference value; generating, by the determination result providing system, input data based on the first staining intensity reference value to the N^{th} staining intensity reference value and the first feature vector to the N^{th} feature vector of the determination target immunohistochemically stained image; and outputting, by the determination result providing system, a determination result for the determination target pathological specimen determined by the machine learning model based on the input data, and wherein the generating of the n^{th} feature vector of the determination target immunohistochemically stained image based on the staining intensity for each pixel of the determination target immunohistochemically stained image and the n^{th} staining intensity reference value includes generating an n^{th} binarized image corresponding to the determination target immunohistochemically stained image by comparing the staining intensity for each pixel of the determination target immunohistochemically stained image with the n^{th} staining intensity reference value, wherein the n^{th} binarized image is an image divided into an area having a staining intensity greater than the n^{th} staining intensity reference value and an area not having the same, and generating the n^{th} feature vector of the determination target immunohistochemically stained image based on the n^{th} binarized image corresponding to the determination target immunohistochemically stained image.

### Advantageous Effects

According to the technical idea of the present disclosure, it is possible to provide a method and a system capable of precisely analyzing the expression level of a biomarker by analyzing an IHC stained tissue slide using deep learning. In other words, it is possible to provide a method and a system capable of performing determination on various pathological specimen images, such as training a machine learning model used to precisely analyze the expression level of a biomarker and analyzing the expression level of the biomarker through the machine learning model.

A machine learning model training method according to the technical idea of the present disclosure has the characteristic of applying multiple different staining intensity reference values to a single IHC stained image and constructing training data using values derived therefrom, thereby having an effect of enabling the construction of a machine learning model capable of precisely analyzing the expression level of a biomarker.

### Brief Description of Drawings

In order to more fully understand the drawings cited in the detailed description of the present disclosure, a brief description of each drawing is provided.
FIG. 1 is a diagram schematically illustrating an environment in which a machine learning model training method and a determination result providing method for a pathological specimen are performed in accordance with a technical idea of the present disclosure.
FIG. 2 is a flowchart for explaining a machine learning model training method in accordance with an embodiment of the present disclosure.
FIG. 3A illustrates a pathological image stained with a dye in which an immunohistochemical staining reagent and a counterstaining reagent are mixed, and FIG. 3B is a diagram illustrating an immunohistochemically stained image in which immunohistochemical staining colors are extracted from FIG. 3A.
FIG. 4 is a diagram specifically illustrating an example of step S140 of FIG. 2.
FIG. 5 is a diagram illustrating a result of a binarized image changing as a staining intensity reference value increases.
FIG. 6 is a flowchart illustrating an example of a determination result providing method for a pathological specimen in accordance with an embodiment of the present disclosure.
FIG. 7 is a diagram illustrating a schematic configuration of a machine learning model training system in accordance with an embodiment of the present disclosure, and FIG. 8 is a diagram illustrating a schematic configuration of a determination result providing system in accordance with an embodiment of the present disclosure.

### Best Mode for Carrying Out the Invention

Since the present disclosure may apply various transformations and have various embodiments, specific embodiments will be illustrated in the drawings and described in detail in the detailed description. However, this is not intended to limit the present disclosure to specific embodiments, and it should be understood to include all transformations, equivalents and substitutes included in the spirit and scope of the present disclosure. In describing the present invention, if it is determined that a detailed description of related known technologies may obscure the gist of the present disclosure, the detailed description will be omitted.

Terms such as first and second may be used to describe various components, but the components should not be limited by the terms. Terms such as first and second do not imply any particular order and are used only for the purpose of distinguishing one component from another.

The terms used in the present application are used only to describe a particular embodiment and are not intended to limit the present disclosure. Singular expressions include plural expressions unless the context clearly means otherwise.

In this specification, terms such as "comprise", "include" or "have" are intended to designate the presence of features, numbers, steps, operations, components, parts, or combinations thereof described in the specification, and it should be understood that it does not preclude the possibility of the presence or addition of one or more other features, numbers, steps, operations, components, parts, or combinations thereof.

In addition, in the present specification, when one component 'transmits' data to another component, it means that the component may directly transmit the data to the other component, and that the data may be transmitted to the other component through at least one other component. Conversely, when one component 'directly transmits' data to another component, it means that the data is transmitted from the component to the other component without going through the other component.

Hereinafter, with reference to the accompanying drawings, the present disclosure will be described in detail centering on embodiments of the present disclosure. Like reference numerals in each figure indicate like members.

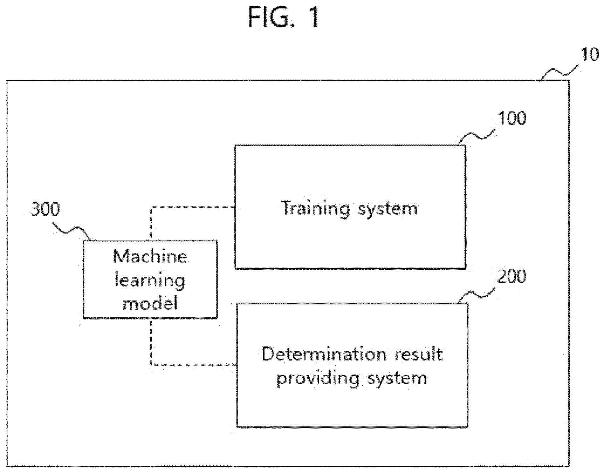

FIG. 1 is a diagram schematically illustrating an environment in which a machine learning model training method and a determination result providing method for a pathological specimen are performed in accordance with a technical idea of the present disclosure.

Referring to FIG. 1, a machine learning model training method according to an embodiment of the present disclosure may be performed by a machine learning model training system 100 (hereinafter, also referred to as a 'training system'), and a determination result providing system for a pathological specimen according to an embodiment of the present disclosure may be performed by a determination result providing system for a pathological specimen 200 (hereinafter, also referred to as a 'determination result providing system').

The training system 100 may receive data of a predetermined format derived from the staining intensity of tissue (e.g., cell membrane or cell nucleus, etc.) stained by IHC staining method, and train a machine learning model 300 to provide the expression level of a predetermined biomarker, detection of lesions included in a pathological specimen, output of diagnostic information for a pathological specimen, or prognostic information and/or response information for a treatment method, and the determination result providing system 200 may make various determinations on the target specimen (e.g., determination on the expression level of a predetermined biomarker, detection of lesions, determination on presence or absence of disease expression, prognosis, treatment method, etc.) using the trained machine learning model 300.

The training system 100 and/or the determination result providing system 200 may be a computing system, which is a data processing device having a computational ability for implementing the technical idea of the present disclosure, and may generally include a server, which is a data processing device that may be accessed by a client through a network, as well as a computing device such as a personal computer or a mobile terminal.

The training system 100 and/or the determination result providing system 200 may be implemented by any single physical device, but an average expert in the technical field of the present disclosure will be able to easily infer that a plurality of physical devices may be organically combined as needed to implement the training system 100 and/or the determination result providing system 200 according to the technical idea of the present disclosure.

The training system 100 may train the machine learning model 300 based on training data generated from a plurality of immunohistochemically stained images.

The immunohistochemically stained image may be an image of a pathological specimen slide stained by a predetermined IHC staining method or a part thereof. The pathological specimen may be a biopsy collected from various organs of the human body or a biological tissue excised through surgery. Meanwhile, the immunohistochemically stained image may be a tissue slide image in which a slide of the pathological specimen is stained by an immunohistochemical staining reagent, or a tissue slide image stained by a dye in which an immunohistochemical staining reagent and a counterstaining reagent are mixed. At this time, the immunohistochemical staining reagent may be a dye in the form of a combination of an antibody targeting a specific biomarker and a chromogenic agent such as diaminobenzidine (DAB), and the counterstaining reagent may be a hematoxylin staining reagent (hereinafter referred to as 'H reagent').

Meanwhile, the immunohistochemically stained image may be a digital slide image of a stained pathological specimen or a part of a digital slide image. The slide of the pathological specimen may be a part of a sliced pathological specimen. The digital slide image of a pathological specimen may be created by slicing a pathological specimen to create a glass slide, staining it with a predetermined dye, and digitizing it. In other words, the immunohistochemically stained image may be a pathological slide image obtained by preparing a pathological specimen as a slide and staining it with a predetermined dye (for example, an immunohistochemical dye or a dye in which an immunohistochemical staining and a counterstaining reagents are mixed), or an image obtained by dividing the stained pathological slide image into a predetermined size.

The training system 100 may generate individual training data using the digital pathological image of the pathological specimen and input the data into an input layer of the machine learning model 300 to train the machine learning model 300. The process by which the training system 100 generates training data will be described later.

The machine learning model 300 may be, but is not limited to, an artificial neural network or a deep neural network and may include various models that may be trained through training data. For example, the machine learning model 300 may be a support vector machine, a random forest, a gradient boosting tree, or an ensemble model thereof.

In addition, the learning of the machine learning model may be supervised learning. For example, if the machine learning model 300 is a deep neural network, the training of the machine learning model 300 may be performed by a back propagation method that updates the weights of the machine learning model 300 so that the loss value, which is the difference between the predicted value output by the machine learning model that receives the training data and the actual value labeled in the training data, is minimized, and at this time, a technique such as the gradient descent method may be used. Since the details of the training process of the machine learning model were widely known at the time the present disclosure was filed, a more detailed description will be omitted.

The machine learning model 300 may be a machine learning model trained to output a probability value for the expression level of a specific biomarker. For example, the machine learning model 300 may output a value representing the expression level of human epidermal growth factor receptor 2 (HER2), estrogen receptor (ER), and/or progesterone receptor (PR). However, the technical idea of the present disclosure is not limited thereto, and a numerical value representing a determination result for a target specimen (e.g., the possibility of disease expression), i.e., a probability value, may be output, and the output value may vary depending on a label tagged to the training data. According to embodiments, the machine learning model 300 may be a machine learning model trained to output probability values for the occurrence of a disease, treatment method, or effect of a specific treatment method for a predetermined disease.

In an embodiment, the machine learning model 300 may be a patch level classification neural network. The patch level classification neural network may be a neural network that receives an image in patch units as an input and outputs a value for classifying the corresponding patch. In another embodiment, the machine learning model 300 may be a pixel level classification neural network. The pixel level classification neural network may be a neural network that outputs a value for classifying each pixel included in the image.

In the present specification, an artificial neural network is a machine learning model artificially constructed based on the operating principles of human neurons, including a multilayer perceptron model, and may mean a set of information expressing a series of design specifications defining an artificial neural network. In an embodiment, the machine learning model 300 may be a convolutional neural network or may include a convolutional neural network.

Meanwhile, the trained machine learning model 300 may be stored in the determination result providing system 200, and the determination result providing system 200 may use the trained machine learning model to make a determination on a predetermined diagnostic target specimen.

As illustrated in FIG. 1, the training system 100 and/or the determination result providing system 200 may be implemented in the form of a subsystem of a predetermined parent system 10. The parent system 10 may be a server. The server 10 means a data processing device having a computational capability for implementing the technical idea of the present disclosure, and generally, not only a data processing device that may be accessed by a client through a network, but also any device capable of performing a specific service, such as a personal computer or a mobile terminal, may be defined as a server, which an average expert in the technical field of the present disclosure will be able to easily infer.

Alternatively, according to embodiments, the training system 100 and the determination result providing system 200 may be implemented in separate forms.

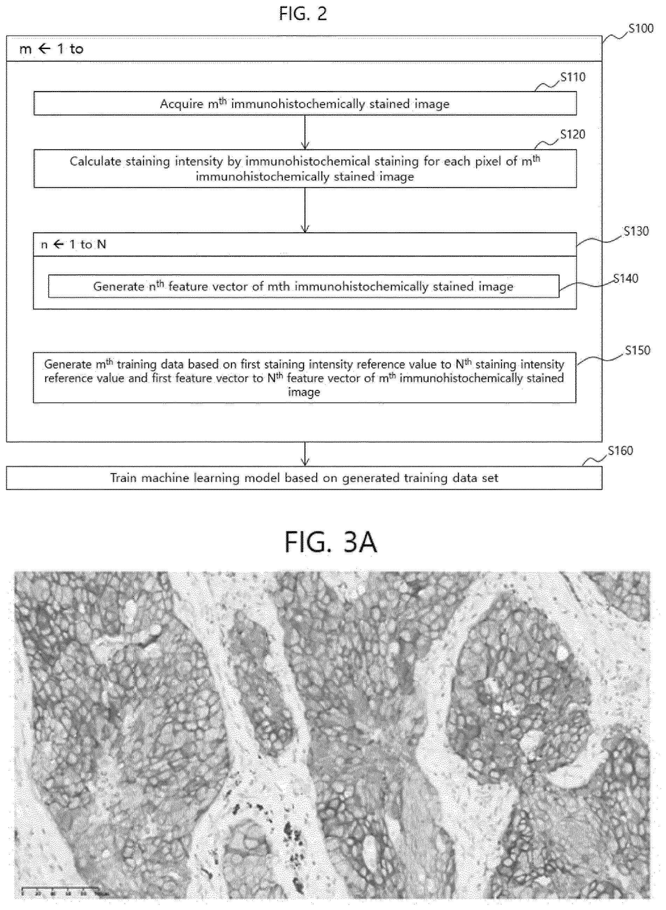

FIG. 2 is a flowchart for explaining a machine learning model training method in accordance with an embodiment of the present disclosure.

Referring to FIG. 2, the training system 100 may generate a training data set including M pieces of individual training data (where M is a natural number of 2 or more). To this end, the training system 100 may generate, for all integers m where 1<=m<=M, m^{th} training data to be included in the training data set (S100).

In order to generate training data, the training system 100 may acquire an m^{th} immunohistochemically stained image (S 110). Here, the immunohistochemically stained image may include an area corresponding to an immunohistochemically stained tissue stained by the IHC staining method for staining a predetermined target biomarker.

In an embodiment, the m^{th} immunohistochemically stained image may be a pathological slide image (or patch) stained with a diaminobenzidine (DAB) reagent.

According to embodiments, the training system 100 may acquire an m^{th} original pathological image generated by scanning a pathological slide stained with a dye in which an immunohistochemical staining reagent (e.g., DAB reagent) and a counterstaining reagent (e.g., H reagent) are mixed, and may separate an immunohistochemically stained part stained with the immunohistochemical staining reagent and a counterstained part stained with the counterstaining reagent from the m^{th} original pathological image, thereby generating the m^{th} immunohistochemically stained image corresponding to the m^{th} original pathological image and an m^{th} counterstained image corresponding to the m^{th} original pathological image.

There may be various methods for separating the immunohistochemically stained part and counterstained part from the original pathological image stained with a dye in which an immunohistochemical staining reagent (e.g., DAB reagent) and a counterstaining reagent (e.g., H reagent) are mixed, and one example is a method using color deconvolution (Quantification of histochemical staining by color deconvolution; Anal Quant Cytol Histol 23: 291-299, 2001.). The present method is roughly a method that converts the signal intensity of each channel in the color space expressing the original pathological image into an optical density, and then converts the optical density into a staining intensity according to a predetermined correlation formula (which is determined experimentally), and separates the immunohistochemically stained part and the counterstained part based on whether it is greater than or less than a specific staining intensity.

In an embodiment, the training system 100 may receive the m^{th} immunohistochemically stained image or m^{th} original pathological image corresponding to a predetermined pathological specimen from an external terminal, and in another embodiment, the training system 100 may also acquire from a memory device storing the m^{th} immunohistochemically stained image or m^{th} original pathological image corresponding to a pathological specimen in advance.

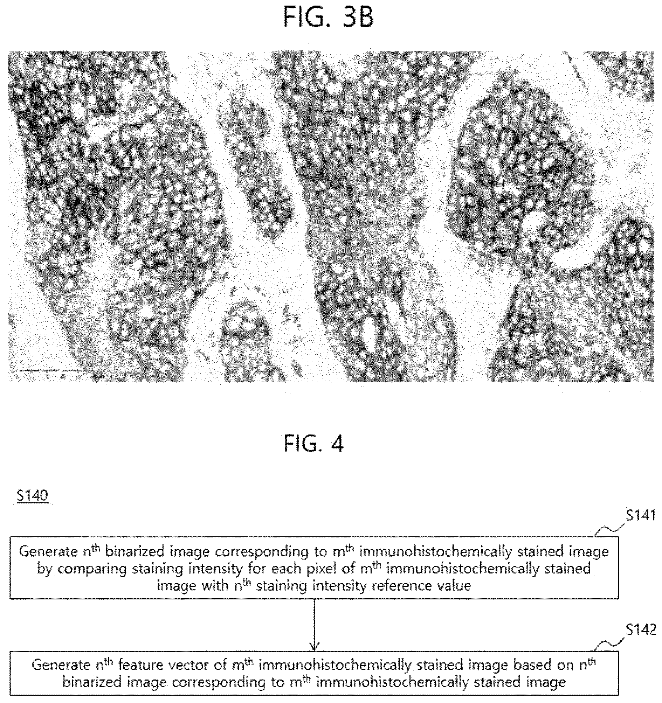

FIG. 3A illustrates a pathological image stained with a dye in which an immunohistochemical staining reagent and a counterstaining reagent are mixed, and FIG. 3B is a diagram illustrating an immunohistochemically stained image in which immunohistochemical staining colors are extracted from FIG. 3A.

Meanwhile, the m^{th} immunohistochemically stained image or the m^{th} original pathological image may be labeled with certain information, and the machine learning model 300 may be trained by a supervised learning method. The labeled information may vary depending on the purpose of the machine learning model 300. For example, the information labeled in the m^{th} immunohistochemically stained image or the m^{th} original pathological image may be the expression level of HER2, the proportion score and/or the staining intensity score of the immunohistochemically stained part, or the ER, PR expression level score, but the technical idea of the present disclosure is not limited thereto. According to embodiments, the present disclosure may be used to create new biomarker expression level reading rules that did not previously exist. In particular, by using a method that trains to increase the correlation between the output values and the actual treatment effect using linear regression, logistic regression, and other machine learning models, it is possible to discover new biomarker expression level reading rules that are very effective in determining the treatment method.

Meanwhile, the training system 100 may calculate the staining intensity by immunohistochemical staining for each pixel of the m^{th} immunohistochemically stained image (S120). In an embodiment, the training system 100 may calculate the staining intensity of each pixel converted from the optical density determined in the color deconvolution method described above as the staining intensity for each pixel of the m^{th} immunohistochemically stained image. Alternatively, the training system 100 may convert the m^{th} immunohistochemically stained image into a black and white image and use the brightness of the converted black and white image to calculate the staining intensity for each pixel of the m^{th} immunohistochemically stained image.

Thereafter, the training system 100 may generate N different feature vectors (S130). More specifically, the training system 100 may generate, for all integers n where 1<=n<=N (where N is an integer of 2 or more), an n^{th} feature vector of the m^{th} immunohistochemically stained image based on the staining intensity for each pixel of the m^{th} immunohistochemically stained image and a predetermined n^{th} staining intensity reference value (S140). At this time, for all integers i where 1<=i<=(N-1), an i^{th} staining intensity reference value may be smaller than an (i+1)^{th} staining intensity reference value. At this time, the staining intensity reference values are not equal to each other, and when 1 <= i < j <= N, the reference value t_i < tj may be satisfied. In other words, the N staining intensity reference values may be values which gradually increase. Meanwhile, the staining intensity reference values may be determined by constructing image data to include various staining intensities and considering the distribution of staining intensity values measured from the constructed data, or may be determined by other methods.

Meanwhile, the above vector generation method may be implemented by applying one or more staining intensity reference values to the m^{th} immunohistochemically stained image, determining a part having a staining intensity exceeding the reference value as stained, and generating a binarized image corresponding to each reference value, the specific method of which is illustrated in FIG. 4. FIG. 4 is a diagram specifically illustrating an example of step S 140 of FIG. 2.

Referring to FIG. 4, the training system 100 may generate an n^{th} binarized image corresponding to the m^{th} immunohistochemically stained image by comparing the staining intensity for each pixel of the m^{th} immunohistochemically stained image with the n^{th} staining intensity reference value (S141). At this time, the n^{th} binarized image is an image divided into an area having a staining intensity greater than the n^{th} staining intensity reference value and an area not having the same. For example, the training system 100 may generate the binarized image by assigning a value of a pixel having a staining intensity greater than the n^{th} staining intensity reference value to 1 and assigning a value of a pixel having a staining intensity less than the n^{th} staining intensity reference value to 0.

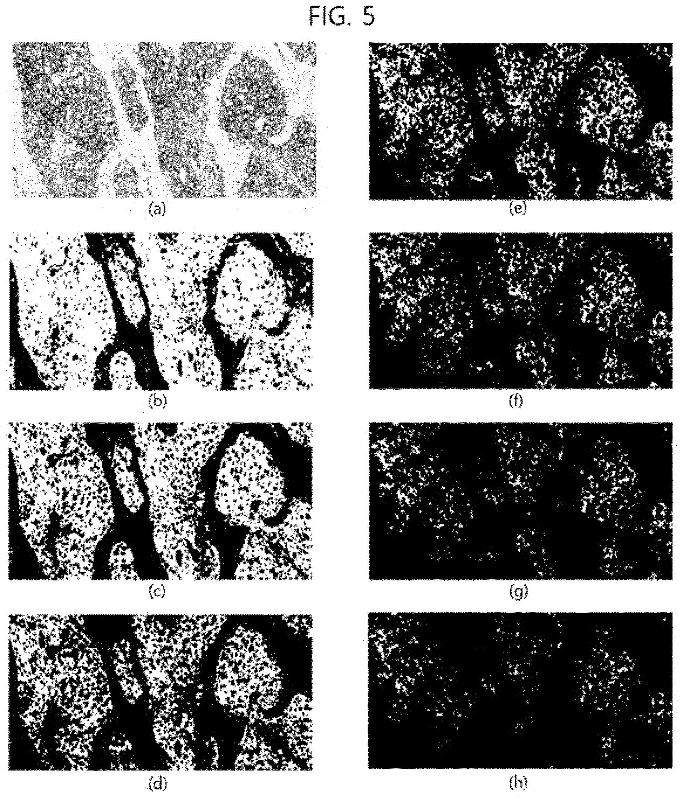

FIG. 5 is a diagram illustrating a result of a binarized image changing as a staining intensity reference value increases. FIG. 5A illustrates an immunohistochemically stained image, and FIGS. 5B to 5H illustrate binarized images corresponding to the first staining intensity reference value to seventh staining intensity reference value, respectively, and it may be seen that the binarized images change as the staining intensity reference value increases.

Referring again to FIG. 4, the training system 100 may generate the n^{th} feature vector of the m^{th} immunohistochemically stained image based on the n^{th} binarized image corresponding to the m^{th} immunohistochemically stained image (S142). The feature vector may be composed of 1 or 2 or more components. The fact that a feature vector is generated based on a binarized image means that one of the main factors in determining the feature vector is the binarized image, and other factors may be additionally used in the process of generating the feature vector in addition to the binarized image.

In an embodiment, the training system 100 may generate the n^{th} feature vector of the m^{th} immunohistochemically stained image, in which each of at least one calculated value calculated based on the n^{th} binarized image corresponding to the m^{th} immunohistochemically stained image and the m^{th} counterstained image is a component of the vector. At this time, the at least one calculated value may include at least one of a proportion of stained cell tissue, a proportion of cells with stained cell membranes, and a proportion of cells with stained cell nuclei, and the proportion of cells with stained cell membranes may include a proportion of cells with completely stained cell membranes and a proportion of cells with partially stained cell membranes.

For example, the training system 100 may calculate the ratio of the area of pixels having a value of '1' (i.e., pixels having a staining intensity greater than the n^{th} staining intensity reference value) in the n^{th} binarized image and the area of stained pixels in the m^{th} counterstained image to derive the ratio of stained cell tissue and determine it as one of the components of the n^{th} feature vector. Alternatively, the training system 100 may determine a closed area determined by pixels having a value of '1' in the n^{th} binarized image (i.e., pixels having a staining intensity greater than the n^{th} staining intensity reference value), calculate a ratio of the area occupied by the closed area and the area of stained pixels in the m^{th} counterstained image to derive a ratio of cells with stained cell membranes, and determine it as one of the components of the n^{th} feature vector. Alternatively, the training system 100 may determine a closed area determined by pixels having a value of '1' in the n^{th} binarized image (i.e., pixels having a staining intensity greater than the n^{th} staining intensity reference value), calculate a ratio of cells with completely stained cell membranes based on an area of cells surrounded by an outline in which a ratio of pixels having a value of '1' in the n^{th} binarized image is greater than a certain size among the outlines of each closed area, and determine it as one of the components of the n^{th} feature vector. In addition to this, the components of the feature vector may be determined in various ways.

Referring to FIG. 2 again, the training system 100 may generate the m^{th} training data based on the first staining intensity reference value to N^{th} staining intensity reference value and the first feature vector to N^{th} feature vector of the m^{th} immunohistochemically stained image (S150).

In an embodiment, the m^{th} training data may consist of a pair of the first staining intensity reference value and the first feature vector to a pair of the N^{th} staining intensity reference value and the N^{th} feature vector. For example, the m^{th} training data may be [<t_1, F_1>, <t_2, F_2>, ..., <t_N, F_N>] (t_i is the i^{th} staining intensity reference value, and F_i is the i^{th} feature vector of the m^{th} immunohistochemically stained image).

Alternatively, in another embodiment, the m^{th} training data may consist of a pair of the first staining intensity reference value and a first feature vector difference value to a pair of an (N-1)^{th} staining intensity reference value and an (N-1)^{th} feature vector difference value. For example, the m^{th} training data may be [<t_1, F_2-F1>, <t_2, F_3-F_2>, ..., <t_(N-1), F_N-F_(N-1)>].

In addition, each training data may be labeled with a value representing the expression level reading rule of the biomarker. The values labeled for each training data may be information pre-tagged to the m^{th} immunohistochemically stained image or the m^{th} original pathological image, or information derived from such information.

For example, each training data may be labeled with an expression level score of HER2, an expression score of estrogen receptor (ER), progesterone receptor (PR), a proportion score of stained tissue, and/or a staining intensity score. However, the labels listed above are only examples, and if there is diagnostic information, prognostic information, and/or response information for a specific treatment method for a pathological specimen corresponding to the m^{th} training data, the training system 100 may set this as a label of the training data. In addition, various values representing new biomarker expression level reading rules that did not previously exist may be labeled. For example, values such as treatment effect or expected life expectancy after surgery may be labeled, or predetermined values designed to increase the correlation between the output values from linear regression, logistic regression, or other machine learning models and the actual treatment effect may be labeled.

When a training data set including M pieces of individual training data is generated through the above method, the training system 100 may input the generated training data set into the input layer of the machine learning model 300 to train the machine learning model 300 (S160 of FIG. 2). Machine learning processes such as deep learning techniques that input training data tagged with correct answer information into the machine learning model 300 and train the machine learning model 300 through methods such as gradient descent and back propagation are widely known, so a detailed description will be omitted.

As discussed above, the machine learning model training method according to the technical idea of the present disclosure has the characteristic of applying multiple different staining intensity reference values to a single immunohistochemically stained image and organizing training data using the values derived therefrom, and through this, has the effect of enabling the construction of a machine learning model that may precisely analyze the expression level of biomarkers.

The method of training machine learning models by organizing training data as described above may be applied in various ways. In an embodiment, the above training method may be used to receive input values based on [<t_i, F_i>] generated by extracting the feature vector F_i including the proportion of cells with completely stained cell membranes among cancer cells for the plurality of different staining intensity reference values t_i, train the machine learning model that outputs the HER2 expression level score, and determine the expression level of HER2 through the trained model.

Alternatively, it may be used to receive input values based on [( t_i, F_(i+1) - F_i)] derivable from [<t_i, F_i>] by extracting the feature vector F_i including the proportion of cells with stained nuclei for the plurality of different staining intensity reference values t_i, train the machine learning model that outputs the proportion score and intensity score, and actually determine the proportion score and intensity score through the trained model.

It may also be used to create new biomarker expression level reading rules that did not previously exist. In particular, by using a method that trains to increase the correlation between the output values and the actual treatment effect using linear regression, logistic regression, and other machine learning models, it is possible to discover new biomarker expression level reading rules that are very effective in determining the treatment method.

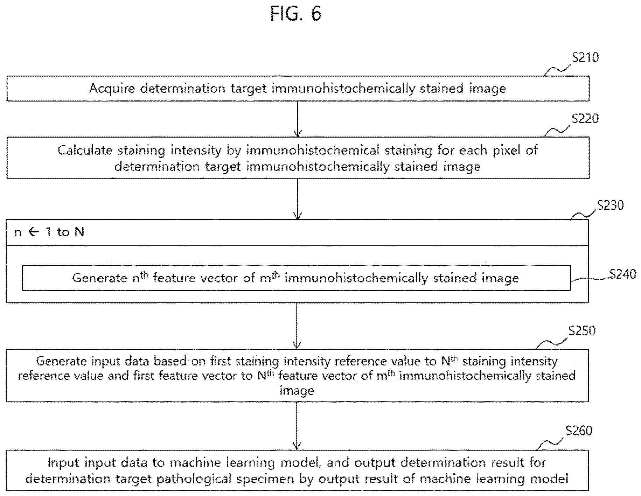

FIG. 6 is a flowchart illustrating an example of a determination result providing method for a pathological specimen in accordance with an embodiment of the present disclosure. The determination result providing method for a pathological specimen according to FIG. 6 may be performed by the determination result providing system 200, and the determination result providing system 200 may store the machine learning model 300 trained by the training system 100.

Referring to FIG. 6, the determination result providing system 200 may acquire a determination target immunohistochemically stained image of a predetermined determination target pathological specimen (S210). At this time, the determination target immunohistochemically stained image may include an area corresponding to the immunohistochemically stainied tissue of the determination target pathological specimen stained by the IHC staining method.

The determination result providing system 200 may calculate the staining intensity by immunohistochemical staining for each pixel of the determination target immunohistochemically stained image (S220), generate the first feature vector to the Nth feature vector from the determination target immunohistochemically stained image (S230, S240), and generate input data based on the first staining intensity reference value to N^{th} staining intensity reference value and the first feature vector to N^{th} feature vector of the determination target immunohistochemically stained image (S250). The process of generating input data corresponding to the immunohistochemical image of the determination target specimen is very similar to the process described above, so a separate description will be omitted.

The determination result providing system 200 may input input data into the machine learning model 300 and output the result output by the machine learning model or output the determination result for the determination target pathological specimen based on the result output by the machine learning model S260.

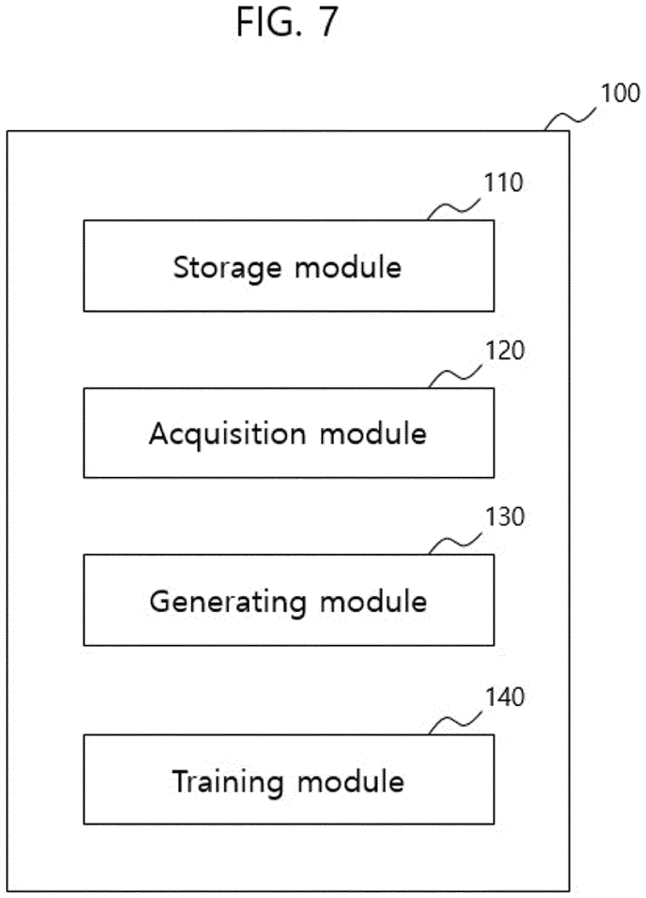

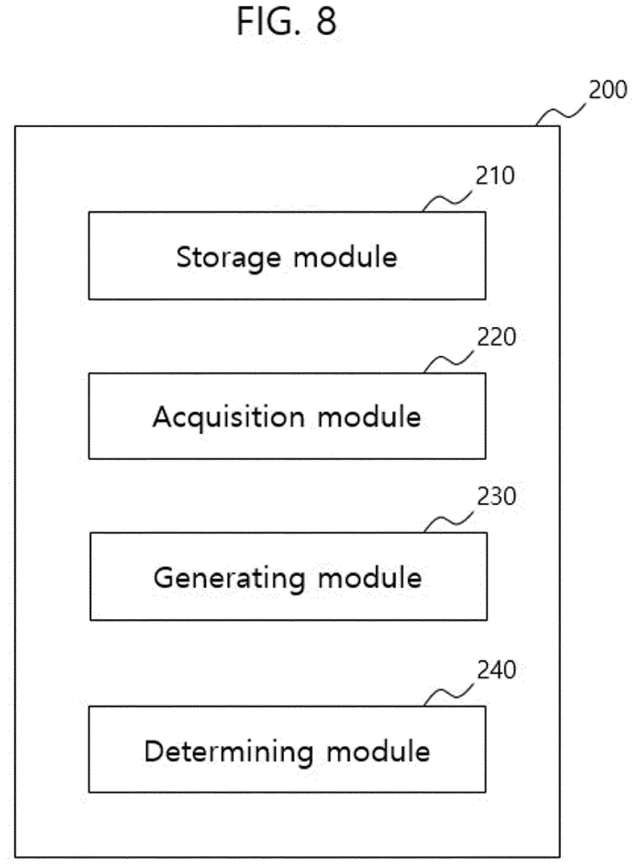

FIG. 7 is a diagram illustrating a schematic configuration of a machine learning model training system 100 in accordance with an embodiment of the present disclosure, and FIG. 8 is a diagram illustrating a schematic configuration of a determination result providing system 200 in accordance with an embodiment of the present disclosure.

The machine learning model training system 100 and the determination result providing system 200 may mean a logical configuration equipped with hardware resources and/or software necessary to implement the technical idea of the present disclosure, and do not necessarily mean one physical component or one device. In other words, the machine learning model training system 100 and the determination result providing system 200 may mean a logical combination of hardware and/or software provided to implement the technical idea of the present disclosure, and, if necessary, may be implemented as a set of logical configurations for implementing the technical idea of the present disclosure by being installed in devices spaced apart from each other and performing each function. In addition, the machine learning model training system 100 and the determination result providing system 200 may mean a set of configurations separately implemented for each function or role to implement the technical idea of the present disclosure. Each configuration of the machine learning model training system 100 and the determination result providing system 200 may be located in different physical devices or may be located in the same physical device. In addition, according to implementation examples, the combination of software and/or hardware constituting each component of the machine learning model training system 100 and the determination result providing system 200 may also be located in different physical devices, and the configurations located in different physical devices may be organically combined with each other to implement each of the modules.

In addition, the term "module" in the present specification may mean a functional and structural combination of hardware for performing the technical idea of the present disclosure and software for operating the hardware. For example, it may be easily inferred by an average expert in the technical field of the present disclosure that the module may mean a logical unit of a given code and hardware resources for executing the given code, and does not necessarily mean physically connected code or a type of hardware.

Referring to FIG. 7, the machine learning model training system 100 may include a storage module 110, an acquisition module 120, a generating module 130, and a training module 140. According to embodiments of the present disclosure, some of the components among the components described above may not necessarily correspond to components essential to the implementation of the present disclosure, and according to embodiments, the machine learning model training system 100 may include more components. For example, the machine learning model training system 100 may further include a communication module (not shown) for communicating with an external device and a control module (not shown) for controlling components and resources of the machine learning model training system 100.

The above storage module 110 may store a machine learning model 300 to be trained.

The acquisition module 120 may acquire M pieces of immunohistochemically stained images including areas corresponding to immunohistochemically stained tissues stained by the IHC staining method for staining a predetermined target biomarker. In an embodiment, the acquisition module may acquire an original pathological image generated by scanning a pathological slide stained with a dye in which an immunohistochemical staining reagent and a counterstaining reagent are mixed, and may separate an immunohistochemically stained part stained with the immunohistochemical staining reagent and a counterstained part stained with the counterstaining reagent from the original pathological image, thereby generating the immunohistochemically stained image corresponding to the original pathological image and a counterstained image corresponding to the original pathological image.

The generating module 130 may generate individual training data based on each immunohistochemically stained image and predetermined staining intensity reference values, and may configure a training data set including a plurality of individual training data. The individual training data generated by the generating module 130 may be a vector expressing the expression level of a biomarker.

The training module 140 may train the machine learning model 300 based on the training data set.

Referring to FIG. 8, the determination result providing system 200 may include a storage module 210, an acquisition module 220, a generating module 230, and a determining module 240. According to embodiments of the present disclosure, some of the components among the components described above may not necessarily correspond to components essential to the implementation of the present disclosure, and according to embodiments, the determination result providing system 200 may include more components. For example, the determination result providing system 200 may further include a communication module (not shown) for communicating with an external device, and a control module (not shown) for controlling components and resources of the determination result providing system 200.

The storage module 210 may store a trained machine learning model 300.

The acquisition module 220 may acquire a determination target immunohistochemically stained image.

The generating module 230 may generate input data based on the determination target immunohistochemically stained image.

The determining module 240 may input the input data into the machine learning model 300 and perform a determination on the determination target specimen based on the predicted value output from the machine learning model 300.

Meanwhile, according to implementation examples, the machine learning model training system 100 and the determination result providing system 200 may include a processor and a memory that stores a program executed by the processor. The processor may include a single core CPU or a multi-core CPU. The memory may include high-speed random access memory and may also include non-volatile memory, such as one or more magnetic disk storage devices, flash memory devices, or other non-volatile solid-state memory devices. Access to the memory by the processor and other components may be controlled by a memory controller.

Meanwhile, the method according to an embodiment of the present disclosure may be implemented in the form of computer-readable program instructions and stored in a non-transitory computer-readable recording medium, and the control program and target program according to an embodiment of the present disclosure may also be stored in a non-transitory computer-readable recording medium. A non-transitory computer-readable recording medium includes all types of recording devices in which data that may be read by a computer system is stored.

Program instructions recorded on the recording medium may be those specifically designed and configured for the present disclosure, or may be known and available to those skilled in the software field.

Examples of the non-transitory computer-readable recording medium include magnetic media such as a hard disk, a floppy disk, and a magnetic tape, optical media such as a CD-ROM and a DVD, magneto-optical media such as a floptical disk, and hardware devices specially configured to store and perform program instructions such as a ROM, a RAM, and a flash memory. In addition, the non-transitory computer-readable recording medium is distributed in computer systems connected through a network, so that computer-readable codes may be stored and executed in a distributed manner.

Examples of program instructions include not only machine language code such as that created by a compiler, but also high-level language code that may be executed by a device that electronically processes information using an interpreter, for example, a computer.

The hardware devices described above may be configured to operate as one or more software modules to perform the operations of the present disclosure, and vice versa.

The above description of the present disclosure is for illustrative purposes, and those skilled in the art to which the present disclosure pertains will understand that the present disclosure may be easily modified into other specific forms without changing the technical idea or essential features of the present disclosure. Therefore, the embodiments described above should be understood in all respects as illustrative and not restrictive. For example, each component described as unitary may be implemented in a distributed manner, and similarly, components described as distributed may also be implemented in a combined form.

The scope of the present disclosure is indicated by the appended claims rather than the detailed description above, and all changes or modified forms derived from the meaning and scope of the claims and their equivalent concepts should be construed as being included in the scope of the present disclosure.

### Industrial Applicability

The present disclosure may be used in a method for training a machine learning model for analyzing an immunohistochemically stained image and a computing system for performing the same.

## Claims

1. A method for training a machine learning model, the method comprising:
generating, by a machine learning model training system, a training data set including M pieces of individual training data (where M is a natural number of 2 or more); and
training, by the machine learning model training system, the machine learning model based on the training data set,
wherein the generating of the training data set including the M pieces of individual training data comprises, for all integers m where 1<=m<=M, generating m^{th} training data to be included in the training data set,
wherein the generating of the m^{th} training data comprises:
acquiring an m^{th} immunohistochemically stained image, wherein the m^{th} immunohistochemically stained image includes an area corresponding to an immunohistochemically stained tissue stained by an immunohistochemistry (IHC) staining method for staining a predetermined target biomarker;
calculating a staining intensity by immunohistochemical staining for each pixel of the m^{th} immunohistochemically stained image;
for all integers n where 1<=n<=N (where N is an integer of 2 or more), generating an n^{th} feature vector of the m^{th} immunohistochemically stained image based on the staining intensity for each pixel of the m^{th} immunohistochemically stained image and a predetermined n^{th} staining intensity reference value; and
generating the m^{th} training data based on the first staining intensity reference value to the N^{th} staining intensity reference value and the first feature vector to the N^{th} feature vector of the m^{th} immunohistochemically stained image, and
wherein the generating of the n^{th} feature vector of the m^{th} immunohistochemically stained image based on the staining intensity for each pixel of the m^{th} immunohistochemically stained image and the predetermined n^{th} staining intensity reference value comprises:
generating an n^{th} binarized image corresponding to the m^{th} immunohistochemically stained image by comparing the staining intensity for each pixel of the m^{th} immunohistochemically stained image with the n^{th} staining intensity reference value, wherein the n^{th} binarized image is an image divided into an area having a staining intensity greater than the n^{th} staining intensity reference value and an area not having the same, and
generating the n^{th} feature vector of the m^{th} immunohistochemically stained image based on the n^{th} binarized image corresponding to the m^{th} immunohistochemically stained image.

2. The method of claim 1, wherein, for all integers i where 1<=i<=(N-1), the i^{th} staining intensity reference value is smaller than the (i+1)^{th} staining intensity reference value.

3. The method of claim 1, wherein the acquiring of the m^{th} immunohistochemically stained image comprises:
acquiring an m^{th} original pathological image generated by scanning a pathological slide stained with a dye in which an immunohistochemical staining reagent and a counterstaining reagent are mixed; and
separating an immunohistochemically stained part stained with the immunohistochemical staining reagent and a counterstained part stained with the counterstaining reagent from the m^{th} original pathological image, thereby generating the m^{th} immunohistochemically stained image corresponding to the m^{th} original pathological image and an m^{th} counterstained image corresponding to the m^{th} original pathological image.

4. The method of claim 3, wherein the generating of the n^{th} feature vector of the m^{th} immunohistochemically stained image based on the n^{th} binarized image corresponding to the m^{th} immunohistochemically stained image comprises generating the n^{th} feature vector of the m^{th} immunohistochemically stained image, in which each of at least one calculated value calculated based on the n^{th} binarized image corresponding to the m^{th} immunohistochemically stained image and the m^{th} counterstained image is a component of the vector,
wherein the at least one calculated value comprises at least one of a proportion of stained cell tissue, a proportion of cells with stained cell membranes, and a proportion of cells with stained cell nuclei.

5. The method of claim 1, wherein the generating of the m^{th} training data based on the first staining intensity reference value to the N^{th} staining intensity reference value and the first feature vector to the N^{th} feature vector of the m^{th} immunohistochemically stained image comprises generating the m^{th} training data, which comprises a pair of the first staining intensity reference value and the first feature vector to a pair of the N^{th} staining intensity reference value and the N^{th} feature vector, and is labeled with a human epidermal growth factor receptor 2 (HER2) expression score.

6. The method of claim 2, wherein the generating of the m^{th} training data based on the first staining intensity reference value to the N^{th} staining intensity reference value and the first feature vector to the N^{th} feature vector of the m^{th} immunohistochemically stained image comprises generating the m^{th} training data, which comprises a pair of the first staining intensity reference value and a first feature vector difference value to a pair of the (N-1)^{th} staining intensity reference value and an (N-1)^{th} feature vector difference value, and is labeled with an estrogen receptor (ER) or progesterone receptor (PR) expression score,
wherein, for all integers i where 1<=i<=(N-1), the i^{th} feature vector difference value is a difference value between the (i+1)^{th} feature vector and the i^{th} feature vector.

7. A method for providing a determination result for a predetermined determination target pathological specimen through a machine learning model trained by the method according to claim 1, the method comprising:
acquiring, by a computing system, a determination target immunohistochemically stained image, wherein the determination target immunohistochemically stained image includes an area corresponding to an immunohistochemically stained tissue of the determination target pathological specimen stained by the IHC staining method;
calculating, by the computing system, a staining intensity by immunohistochemical staining for each pixel of the determination target immunohistochemically stained image;
for all integers n where 1<=n<=N, generating, by the computing system, an n^{th} feature vector of the determination target immunohistochemically stained image based on the staining intensity for each pixel of the determination target immunohistochemically stained image and the n^{th} staining intensity reference value; and
generating, by the computing system, input data based on the first staining intensity reference value to the N^{th} staining intensity reference value and the first feature vector to the N^{th} feature vector of the determination target immunohistochemically stained image; and
outputting, by the computing system, a determination result for the determination target pathological specimen determined by the machine learning model based on the input data,
wherein the generating of the n^{th} feature vector of the determination target immunohistochemically stained image based on the staining intensity for each pixel of the determination target immunohistochemically stained image and the n^{th} staining intensity reference value comprises:
generating an n^{th} binarized image corresponding to the determination target immunohistochemically stained image by comparing the staining intensity for each pixel of the determination target immunohistochemically stained image with the n^{th} staining intensity reference value, wherein the n^{th} binarized image is an image divided into an area having a staining intensity greater than the n^{th} staining intensity reference value and an area not having the same; and
generating the n^{th} feature vector of the determination target immunohistochemically stained image based on the n^{th} binarized image corresponding to the determination target immunohistochemically stained image.

8. A computer program recorded on a non-transitory medium for performing the method of any one of claims 1 to 7 which is installed in a data processing device.

9. A non-transitory computer-readable recording medium in which a computer program for performing the method of any one of claims 1 to 7.

10. A machine learning model training system comprising:
a processor; and
a memory storing a computer program,
wherein the computer program, when executed by the processor, causes the machine learning model training system to perform a machine learning model training method,
wherein the machine learning model training method comprises:
generating, by the machine learning model training system, a training data set including M pieces of individual training data (where M is a natural number of 2 or more); and
training, by the machine learning model training system, the machine learning model based on the training data set,
wherein the generating of the training data set including the M pieces of individual training data comprises, for all integers m where 1<=m<=M, generating m^{th} training data to be included in the training data set,
wherein the generating of the m^{th} training data comprises:
acquiring an m^{th} immunohistochemically stained image, wherein the m^{th} immunohistochemically stained image includes an area corresponding to an immunohistochemically stained tissue stained by an immunohistochemistry (IHC) staining method for staining a predetermined target biomarker;
calculating a staining intensity by immunohistochemical staining for each pixel of the m^{th} immunohistochemically stained image;
for all integers n where 1<=n<=N (where N is an integer of 2 or more), generating an n^{th} feature vector of the m^{th} immunohistochemically stained image based on the staining intensity for each pixel of the m^{th} immunohistochemically stained image and a predetermined n^{th} staining intensity reference value; and
generating the m^{th} training data based on the first staining intensity reference value to the N^{th} staining intensity reference value and the first feature vector to the N^{th} feature vector of the m^{th} immunohistochemically stained image, and
wherein the generating of the n^{th} feature vector of the m^{th} immunohistochemically stained image based on the staining intensity for each pixel of the m^{th} immunohistochemically stained image and the predetermined n^{th} staining intensity reference value comprises:
generating an n^{th} binarized image corresponding to the m^{th} immunohistochemically stained image by comparing the staining intensity for each pixel of the m^{th} immunohistochemically stained image with the n^{th} staining intensity reference value, wherein the n^{th} binarized image is an image divided into an area having a staining intensity greater than the n^{th} staining intensity reference value and an area not having the same; and
generating the n^{th} feature vector of the m^{th} immunohistochemically stained image based on the n^{th} binarized image corresponding to the m^{th} immunohistochemically stained image.

11. The machine learning model training system of claim 10, wherein,
for all integers i where 1<=i<=(N-1), the i^{th} staining intensity reference value is smaller than the (i+1)^{th} staining intensity reference value.

12. The machine learning model training system of claim 10, wherein the acquiring of the m^{th} immunohistochemically stained image comprises:
acquiring an m^{th} original pathological image generated by scanning a pathological slide stained with a dye in which an immunohistochemical staining reagent and a counterstaining reagent are mixed; and
separating an immunohistochemically stained part stained with the immunohistochemical staining reagent and a counterstained part stained with the counterstaining reagent from the m^{th} original pathological image, thereby generating the m^{th} immunohistochemically stained image corresponding to the m^{th} original pathological image and an m^{th} counterstained image corresponding to the m^{th} original pathological image.

13. The machine learning model training system of claim 12, wherein the generating of the n^{th} feature vector of the m^{th} immunohistochemically stained image based on the n^{th} binarized image corresponding to the m^{th} immunohistochemically stained image comprises generating the n^{th} feature vector of the m^{th} immunohistochemically stained image, in which each of at least one calculated value calculated based on the n^{th} binarized image corresponding to the m^{th} immunohistochemically stained image and the m^{th} counterstained image is a component of the vector,
wherein the at least one calculated value comprises at least one of a proportion of stained cell tissue, a proportion of cells with stained cell membranes, and a proportion of cells with stained cell nuclei.

14. The machine learning model training system of claim 10, wherein the generating of the m^{th} training data based on the first staining intensity reference value to the N^{th} staining intensity reference value and the first feature vector to the N^{th} feature vector of the m^{th} immunohistochemically stained image comprises generating the m^{th} training data, which comprises a pair of the first staining intensity reference value and the first feature vector to a pair of the N^{th} staining intensity reference value and the N^{th} feature vector, and is labeled with a human epidermal growth factor receptor 2 (HER2) expression score.

15. The machine learning model training system of claim 11, wherein the generating of the m^{th} training data based on the first staining intensity reference value to the N^{th} staining intensity reference value and the first feature vector to the N^{th} feature vector of the m^{th} immunohistochemically stained image comprises generating the m^{th} training data, which comprises a pair of the first staining intensity reference value and a first feature vector difference value to a pair of the (N-1)^{th} staining intensity reference value and an (N-1)^{th} feature vector difference value, and is labeled with an estrogen receptor (ER) or progesterone receptor (PR) expression score,
wherein, for all integers i where 1<=i<=(N-1), the i^{th} feature vector difference value is a difference value between the (i+1)^{th} feature vector and the i^{th} feature vector.

16. A determination result providing system for a pathological specimen, the determination result providing system comprising:
a processor; and
a memory storing a computer program,
wherein the computer program, when executed by the processor, causes the determination result providing system to perform a method for providing a determination result for a predetermined determination target pathological specimen through a machine learning model trained by the method according to claim 1,
wherein the method for providing a determination result comprises:
acquiring, by the determination result providing system, a determination target immunohistochemically stained image, wherein the determination target immunohistochemically stained image includes an area corresponding to an immunohistochemically stained tissue of the determination target pathological specimen stained by the IHC staining method;
calculating, by the determination result providing system, a staining intensity by immunohistochemical staining for each pixel of the determination target immunohistochemically stained image;
for all integers n where 1<=n<=N, generating, by the determination result providing system, an n^{th} feature vector of the determination target immunohistochemically stained image based on the staining intensity for each pixel of the determination target immunohistochemically stained image and the n^{th} staining intensity reference value;
generating, by the determination result providing system, input data based on the first staining intensity reference value to the N^{th} staining intensity reference value and the first feature vector to the N^{th} feature vector of the determination target immunohistochemically stained image; and
outputting, by the determination result providing system, a determination result for the determination target pathological specimen determined by the machine learning model based on the input data, and
wherein the generating of the n^{th} feature vector of the determination target immunohistochemically stained image based on the staining intensity for each pixel of the determination target immunohistochemically stained image and the n^{th} staining intensity reference value comprises:
generating an n^{th} binarized image corresponding to the determination target immunohistochemically stained image by comparing the staining intensity for each pixel of the determination target immunohistochemically stained image with the n^{th} staining intensity reference value, wherein the n^{th} binarized image is an image divided into an area having a staining intensity greater than the n^{th} staining intensity reference value and an area not having the same; and
generating the n^{th} feature vector of the determination target immunohistochemically stained image based on the n^{th} binarized image corresponding to the determination target immunohistochemically stained image.
